(19) Europäisches Patentamt
European Patent Office
Office européen des brevets

(11) **EP 4 663 740 A1**

(12) **EUROPEAN PATENT APPLICATION**

(43) Date of publication:
**17.12.2025 Bulletin 2025/51**

(21) Application number: **25177779.3**

(22) Date of filing: **20.05.2025**

(51) International Patent Classification (IPC):
*C12M 1/00* (2006.01)    *C12M 1/107* (2006.01)
*C12M 1/12* (2006.01)    *B01D 53/62* (2006.01)
*B01D 53/84* (2006.01)

(52) Cooperative Patent Classification (CPC):
**C12M 21/02; B01D 53/62; B01D 53/84; C12M 21/04; C12M 23/06**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **13.06.2024 IT 202400013519**

(71) Applicants:
• **Università degli Studi di Napoli Federico II**
  **80125 Napoli (NA) (IT)**
• **Università Telematica Pegaso S.r.l.**
  **80143 Napoli (NA) (IT)**

(72) Inventors:
• **Cesaro, Alessandra**
  **80125 Napoli (IT)**
• **Fabbricino, Massimiliano**
  **80125 Napoli (IT)**
• **Policastro, Grazia**
  **80125 Napoli (IT)**

(74) Representative: **Garavelli, Paolo**
  **A.BRE.MAR. S.R.L.**
  **Consulenza in Proprietà Industriale**
  **Via Servais 27**
  **10146 Torino (IT)**

(54) **PROCESS FOR OPTIMIZING THE PRODUCTION OF MICROALGAL LIPIDS IN A PLANT USED FOR THE SIMULTANEOUS TREATMENT OF LIQUID AND GAS EFFLUENTS FROM ANAEROBIC DIGESTION, AND RELATED PLANT**

(57)     A process and a plant for the production of microalgal lipids with simultaneous treatment of the liquid and gaseous effluents of anaerobic digestion are described; the process includes the steps of: collecting the digestate and separating the liquid fraction from the dry fraction; mixing, within a loading step, the liquid fraction with water and with the reagents necessary to obtain a culture medium suitable for microalgal growth; continuously feeding the culture medium to the mixing step; simultaneously extracting the effluent from the mixing step to the discharge step; activating a recirculation between the mixing step and the absorption step, maintained in the absence of light; simultaneously extracting the effluent as treated biogas; separating the microalgal biomass from the effluent, for subsequent extraction of the lipids; collecting the digestate and separating the liquid fraction from the dry fraction, wherein this step must be implemented upstream of the process.

Fig.1

EP 4 663 740 A1

**Description**

The present invention relates in general to a process for optimizing the production of microalgal lipids in a plant used for the simultaneous treatment of liquid and gaseous effluents from anaerobic digestion, and to the related plant for implementing this process.

The present invention relates in particular to the sector of the production of biofuels from waste and by-products. In detail, it consists in the development of a process for the production of microalgal lipids, precursors of biodiesel, in which, at the same time, the upgrading of the biogas (removal of carbon dioxide) produced by an anaerobic digestion process and the removal of ammoniacal nitrogen from the liquid effluent of the same digestion process take place.

The global challenge of reducing dependence on fossil fuels and fighting climate change through the use of clean and renewable energy sources is a widely debated topic. Currently, in the European Union (EU), the transport and energy sectors represent the two main anthropogenic sources of greenhouse gas emissions. Biofuels are a promising alternative to decarbonize transport and increase energy sustainability. Biofuels are mainly produced from biomass and contribute to lower combustion emissions per unit of equivalent power, compared to fossil fuels. The most common biofuels are biodiesel and bioethanol, which can replace diesel and gasoline, respectively, with little or no modification to existing vehicle engines.

Among the various options for biofuel production, third-generation biorefining plants using microalgae have significant potential. The main advantages of microalgae-derived biofuels include: high growth rates and ease of cultivation.

When grown under optimal physiological conditions, microalgae produce large amounts of biomass, with relatively low lipid content. However, when exposed to stress conditions, microalgae change their biosynthetic pathways from biomass generation to lipid accumulation. For this purpose, different stress conditions have been tested to increase lipid accumulation, such as suboptimal light irradiance, nutrient deficiency, suboptimal culture pH and temperature, and other suboptimal operating conditions. Nitrogen starvation has been shown to be the most widely used technique to stimulate lipid accumulation. Other studies have instead focused on the creation of light-dark cycles. Currently, this technique has been implemented through intermittent illumination of the growth reactor.

To date, processes aimed at accumulating lipids are not yet economically viable. An effective approach to make them profitable consists in integrating them with other processes in order to obtain additional benefits.

In fact, microalgae can be used for different purposes, such as carbon dioxide ($CO_2$) capture and/or wastewater treatment. In particular, it is possible to combine the cultivation of microalgae with the anaerobic digestion process. The latter consists in the transformation of the organic matter present in waste or sewage sludge into a biogas composed mainly of methane and carbon dioxide.

To optimize the efficiency of biogas utilization processes, it is necessary to increase the methane content by eliminating carbon dioxide. Furthermore, the liquid effluent resulting from the process, known as digestate, contains high concentrations of ammonia nitrogen, which is often essential to remove. Previous studies have shown that microalgae are able to remove ammonia nitrogen from the liquid fraction of the digestate. In addition, they can exploit the carbon dioxide contained in the biogas through photosynthetic activity.

Generally, when it comes to biogas upgrading plants with microalgal cultures, the plant configuration is that of the photobioreactor, where the main components are made up of the absorption column for upgrading, the photobioreactor for the growth of microalgae, the algae collection plant and all the accessory components of the plant.

The operation of the absorption column involves the insufflation of the gaseous current from the bottom of the column. The alkaline micro-algal solution present in the column acts as a dispersed medium, and by feeding the gaseous current from the bottom, a significant mixing is guaranteed, increasing the speed of exchange of matter. The absorption column must be shielded, in order to avoid the photosynthetic activity and therefore the production of oxygen within the same plant in which the methane-rich biogas is insufflated. Downstream of the absorption process, the solution is recirculated in the growth photobioreactor, where the microalgae consume the inorganic carbon, due to the photosynthetic activity.

The integration of the lipid accumulation process with the processes of removal of $CO_2$ from the biogas and ammonia nitrogen from the digestate could significantly improve the economic and environmental sustainability of all three processes, since three outputs would be obtained simultaneously. However, the optimal operating conditions for carbon dioxide uptake in the photobioreactor do not coincide with those that maximize lipid accumulation.

In a plant optimized for $CO_2$ uptake, even if the microalgae are subjected to light/dark cycles, the photoperiod is not configured to favour lipid accumulation. Likewise, the plant optimized to induce lipid accumulation is not compatible with the $CO_2$ uptake process.

Therefore, the present invention concerns the simultaneous optimization of both processes, simultaneously feeding the microalgae with anaerobic digestate, so as to obtain the three outputs simultaneously.

The invention relates to a process, as described in claim 1, due to which it is possible to increase the accumulation of microalgal lipids in a reactor plant used to remove carbon dioxide from biogas by means of microalgae. The main innovation concerns the technique used to induce the accumulation of lipids inside the microalgae. This technique consists in subjecting the microalgae to light/dark cycles, through intermittent recirculation inside two spatially distinct components of the reactor: the growth plant, constantly illuminated, and the absorption column, constantly shielded to prevent the entry of light. This plant, compared to the one already tested in previous studies aimed at lipid accumulation (in which the algae are subjected to light and dark cycles using intermittent illumination of a single reactor), has the advantage of being able to integrate the lipid accumulation process, not yet economically sustainable, with both the anaerobic digestion effluent treatment processes (removal of carbon dioxide from the biogas and removal of ammonia nitrogen from the liquid fraction of the digestate). The representative scheme of the process idea is reported below.

This integration can occur through the use of specific operating conditions (HRT, recirculation flow rate, operation and pause times of the recirculation plant, gas flow rate and operation and pause times of the gas injection plant), set in order to simultaneously optimize lipid accumulation, carbon dioxide removal from the biogas and ammonia nitrogen removal from the liquid fraction of the digestate. In order to optimize all three processes simultaneously, the following conditions must be met:

The recirculation of the liquid inside the column must occur with a flow rate such as to ensure that the culture is not exposed to light (dark time in the column) for a period of at least 1.5 consecutive hours/day, that at the same time the ratio between liquid flow rate and gas flow rate is equal to 0.5, the volume of biogas treated is equal to approximately 0.155 L/Lculture/day, the liquid and gas flow rates are such as to overcome the pressure drops in the column, the liquid recirculation pump between the growth reactor and the column must be active for at least 20 minutes before the gas is blown.

The invention also concerns a plant for implementing the above-mentioned process, as specified in the relevant Claim.

Preferred embodiments and non-trivial variants of the present invention form the subject of the dependent claims. It is understood that the attached claims form an integral part of this description.

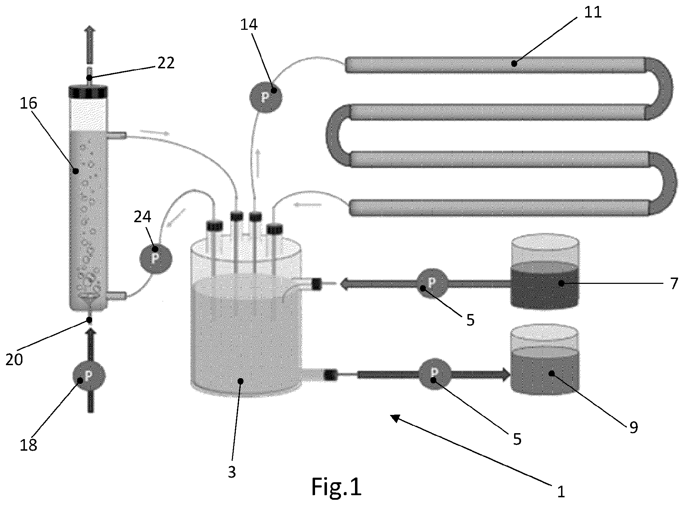

The present invention will be better described by some preferred embodiments, provided by way of example and not limitation, with reference to the attached drawings, in which the only Figure 1 is a schematic representation of a preferred embodiment of the plant of the present invention.

The process of the present invention is used for the production of micro-algal lipids with simultaneous treatment of the liquid and gaseous effluents of anaerobic digestion and includes the steps of:

- collecting the digestate and separating the liquid fraction from the dry fraction. This step must be implemented upstream of the described process. The patent focuses on a process that integrates synergistically with a pre-existing process, anaerobic digestion, which is widely consolidated on an industrial scale. The collection of the digestate and the separation of the solid fraction from the liquid fraction can be performed using any technology already used in anaerobic digestion plants.

- mixing, within a loading step, of the liquid fraction with water and with the reagents necessary to obtain a culture medium suitable for microalgal growth. The culture medium can be a modified Bold's Basal Medium. The modification consists in not inserting the nitrogen source, since this will be supplied through the digestate. Below is a figure showing the composition of the Bold's basal Medium, described in a study by Andersen et al. (2005), in which the modification made is highlighted:

| Sl. No. | Components | Stock Solution (g/L of dH$_2$O) | Quantity Used in BBM | Concentration in Final Medium (M) |
|---|---|---|---|---|
| | *Macronutrients* | | | |
| 1 | NaNO$_3$ | 25.00 | 10 mL | 2.94 × 10$^{-3}$ |
| 2 | CaCl$_2$·2H$_2$O | 2.50 | 10 mL | 1.70 × 10$^{-4}$ |
| 3 | MgSO$_4$·7H$_2$O | 7.50 | 10 mL | 3.04 × 10$^{-4}$ |
| 4 | K$_2$HPO$_4$ | 7.50 | 10 mL | 4.31 × 10$^{-4}$ |
| 5 | KH$_2$PO$_4$ | 17.50 | 10 mL | 1.29 × 10$^{-3}$ |
| 6 | NaCl | 2.50 | 10 mL | 4.28 × 10$^{-4}$ |
| | *Alkaline EDTA Solution* | | 1 mL | |
| 7 | EDTA | 50.00 | | 1.71 × 10$^{-4}$ |
| 8 | KOH | 31.00 | | 5.53 × 10$^{-4}$ |
| | *Acidified Iron Solution* | | 1 mL | |
| 9 | FeSO$_4$·7H$_2$O | 4.98 | | 1.79 × 10$^{-5}$ |
| 10 | H$_2$SO$_4$ | 1 mL | | |
| | *Boron Solution* | | 1 mL | |
| 11 | H$_3$BO$_3$ | 11.42 | | 1.85 × 10$^{-4}$ |
| | *Trace Metals Solution* | | 1 mL | |
| 12 | ZnSO$_4$·7H$_2$O | 8.82 | | 3.07 × 10$^{-5}$ |
| 13 | MnCl$_2$·4H$_2$O | 1.44 | | 7.28 × 10$^{-6}$ |
| 14 | MoO$_3$ | 0.71 | | 4.93 × 10$^{-6}$ |
| 15 | CuSO$_4$·5H$_2$O | 1.57 | | 6.29 × 10$^{-6}$ |
| 16 | Co(NO$_3$)$_2$·6H$_2$O | 0.49 | | 1.68 × 10$^{-6}$ |

The dilution of the digestate must be carried out in such a way as to obtain an ammonia nitrogen concentration of approximately 0.3 moles/L.

Therefore, assuming that the nitrogen concentration in the digestate is equal to x moles/L, it is possible to proceed to the calculation of the dilution factor $f$ to be adopted as follows:

$$f = \frac{x\ moli/L}{0,3\ moli/L} \qquad (1)$$

Consequently, assuming to prepare a volume V of influent (digestate + medium), the volume of digestate to be added will be equal to $\frac{1}{f}V$, while the volume of medium will be equal to $V \cdot \left(1 - \frac{1}{f}\right)$;

- continuous feeding (hydraulic retention time (HRT) of 2.5 days) of the culture medium to the mixing step, and simultaneous extraction of the effluent from the mixing step to the discharge step.

In order to obtain a HRT of 2.5 days, the flow rate Q [L/day], both of influent and effluent, must be calculated as follows:

$$Q = \frac{Vreattore}{HRT} \qquad (2)$$

where **Vreattore** is the total reactor volume;

- activating a recirculation between the mixing step and the absorption step, maintained in the absence of light. The recirculation, carried out by means of a timed pump, must ensure that the crop is not exposed to light (dark time in the column) for a period of at least 1.5 consecutive hours/day, and that at the same time the ratio between liquid flow and gas flow is equal to 0.5. Furthermore, the gas supply must be activated at least 20 minutes after the liquid supply in the absorption column. The gas flow must be such as to ensure that the volume of biogas treated is equal to approximately 0.155 L/Lculture/day. Therefore, the gas flow rate G is determined using the following equation:

$$G = 0,155 \, L/giorno \cdot Vreattore$$

The liquid flow-rate (re-circulation flow-rate) will be equal to:

$$\frac{Qricircolo}{G} = 0,5$$

Since *Qricircolo* must be such as to guarantee the minimum dark time mentioned, it is necessary that the following inequation is verified:

$$Qricircolo \leq \frac{Vcolonna}{HRTcolonna}$$

where *HRTcolonna* is the period of darkness.

In case the calculated flow rates are so low that they cause difficulties in the reactor management, it is possible to adopt a cyclical approach in the liquid and gas supply. This means alternating activation steps with pause steps. In this way, it is possible to increase the liquid and gas flow rates, while maintaining constant the overall daily volumes introduced into the reactor. For example, if the calculated flow rates were respectively 0.5 litres per day for the liquid and 1 litre per day for the gas, but it was necessary to set a liquid flow rate of at least 1 litre per day for management reasons, it would be possible to operate with liquid and gas flow rates of 1 litre per day and 2 litres per day, activating the pumping plants for 12 hours per day and leaving 12 hours of pause. This is possible provided that the minimum HRT (1.5 consecutive hours/day) to be guaranteed in the column is respected. When choosing the activation and pause times and their number, it is important to consider that it is preferable to introduce small doses of biogas into the reactor several times a day rather than supplying the entire amount of carbon necessary for the crop in a single administration. For example, in the case of the previous example of 12 hours of activation followed by 12 hours of pause, it would be preferable to program 6 activation cycles lasting 2 hours each, separated by 6 pause cycles;

- simultaneously extracting the effluent (treated biogas). The extraction of the treated biogas can be carried out using a timed pump, synchronized with the pump used to feed the biogas to be treated;

- separating the micro-algal biomass from the effluent, for subsequent extraction of the lipids. The separation of the biomass can be done using any available technology, for example through centrifugation, filtration, sedimentation or flotation.

As regards the plant of the present invention, it consists of a photobioreactor 1 usable with the process of the present invention and consisting of a mixing chamber (growth reactor) 3 continuously fed by the use of two peristaltic pumps 5 connected to two tanks 7, 9. One tank 7 has been used for loading: this tank 7 must contain the digestate, appropriately diluted. The second tank 9 must have the function of collecting the effluent produced (discharge tank). The mixing chamber (growth reactor) 3 must be connected to a tubular plant 11, composed of transparent tubes and illuminated by LEDs, in order to improve the exposure of the microalgae to light. The recirculation between the growth reactor 3 and the tubular plant 11 must be continuously ensured by a pump 14.

The plant 1 described must be connected to an absorption column 16 by recirculation of the microalgal culture from the mixing chamber 3. For the column 16, a bottom load must be provided, to avoid the sedimentation of the algae, and an "overflow" discharge for the recirculation of the culture from the column to the mixing chamber **3**. The liquid supply in the column 16 must be carried out by a first timed pump 18 (possibility of setting operation-pause intervals).

The column 16 must be equipped with two pipes 20, 22. The first pipe 20, located on the bottom, has the purpose of ensuring the supply of the gas to be treated and must be connected to a diffuser. The second pipe 22, located at the head of column 16, must ensure the exit of the treated gas. The column 16 must be shielded in order to prevent the entry of light and, therefore, the carrying out of the photosynthetic activity by the algae during the absorption process.

The gaseous stream to be treated must be blown by means of a second timed pump 24 (possibility of setting operation-pause intervals).

**Claims**

1.  Process for integrating a production of microalgal lipids with a simultaneous treatment of the liquid and gaseous effluents of anaerobic digestion, said process comprising the steps of:

    - collecting a digestate and separating a liquid fraction from a dry fraction;
    - mixing, within a loading step, the liquid fraction with water and with reagents necessary to obtain a culture medium suitable for microalgal growth;
    - continuously feeding a culture medium to the mixing step;
    - simultaneously extracting the effluent from the mixing step to a discharge step;
    - activating a recirculation between the mixing step and an absorption step, maintained in absence of light, wherein the recirculation of liquid inside a column occurs at a flow rate such as to ensure that the culture is not exposed to light, namely dark time in the column, for a period of at least 1.5 consecutive hours/day, and at the same time the ratio between liquid flow rate and gas flow rate is equal to 0.5, the volume of biogas treated is equal to approximately 0.155 L/Lculture/day, the liquid and gas flow rates are such as to overcome pressure drops in the column, and the liquid recirculation pump between the growth reactor and the column is active for at least 20 minutes before the gas is blown in;
    - simultaneously extracting the effluent as treated biogas;
    - separating the microalgal biomass from the effluent, for subsequent extraction of the lipids;
    - collecting the digestate and separating the liquid fraction from the dry fraction, wherein said step must be implemented upstream of the process.

2.  Process according to claim 1, wherein, in the mixing step, within a loading step, of the liquid fraction with water and with the reagents necessary to obtain a culture medium suitable for microalgal growth, the culture medium is a modified Bold's Basal Medium, wherein the modification consists in not inserting a nitrogen source, since this will be supplied via the digestate.

3.  Process according to claim 2, wherein the dilution of the digestate is carried out in such a way as to obtain an ammonia nitrogen concentration of approximately 0.3 moles/L.

4.  Process according to claim 1, wherein, in the case in which the calculated flow rates are so low as to cause difficulties in managing the reactor, a cyclical approach is adopted in the supply of liquid and gas, i.e. activation steps are alternated with pause steps, in this way it is possible to increase the flow rates of liquid and gas, while maintaining constant the overall daily volumes introduced into the reactor.

5.  Process according to any of the preceding claims, wherein the extraction step of the treated biogas is carried out by means of a timed pump, synchronized with a pump used for the supply of the biogas to be treated.

6.  Process according to any of the preceding claims, wherein the separation step of the biomass occurs using technologies such as centrifugation, filtration, sedimentation or flotation.

7.  Plant for integrating a production of micro-algal lipids with a simultaneous treatment of the liquid and gaseous effluents of anaerobic digestion, said plant being designed to carry out the process according to any of the preceding claims, said plant (1) being of the photobioreactor type and comprising:

    - a mixing chamber or growth reactor (3) continuously fed by two peristaltic pumps (5) connected to two tanks (7, 9), one tank (7) being used for loading and containing the digestate, suitably diluted, and a second tank (9) having the function of collecting the effluent produced, i.e. being a discharge tank;
    - a tubular plant (11) connected to the mixing chamber or growth reactor (3), the tubular plant (11) being composed of transparent tubes and illuminated by LEDs, in order to improve the exposure of the microalgae to light;
    - a pump (14) designed to perform recirculation between the growth reactor (3) and the tubular plant (11);
    - an absorption column (16) connected to the plant (1) by recirculation of the micro-algal culture from the mixing chamber (3), the liquid supply to the column (16) being carried out by means of a first timed pump (18) designed to set operation-pause intervals;
    - two pipes (20, 22) with which the absorption column (16) is equipped, a first pipe (20), being designed to ensure the supply of the gas to be treated and to be connected to a diffuser, and a second pipe (22), positioned at the head of the column (16), must ensure the escape of the treated gas.

8. Plant according to claim 7, wherein the column (16) is shielded in order to avoid the entry of light and, therefore, the performance of the photosynthetic activity by the algae during the absorption process.

9. Plant according to claim 7 or 8, wherein the gaseous stream to be treated is blown in by means of a second timed pump (24) with the possibility of setting operation-pause intervals.

Fig.1

| | Europäisches Patentamt European Patent Office Office européen des brevets | **EUROPEAN SEARCH REPORT** | **Application Number** EP 25 17 7779 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 441 472 A1 (SUKHMEET SINGH ANAND [ES]) 13 February 2019 (2019-02-13) | 1-6 | INV. C12M1/00 |
| Y | * paragraphs [0017], [0018]; claim 1; figure 1 * | 7-9 | C12M1/107 C12M1/12 B01D53/62 |
| Y | DE 39 12 418 C1 (UFZ LEIPZIGHALLE GMBH [DE]) 12 July 1990 (1990-07-12) * paragraph [0002]; claim 2 * | 7-9 | B01D53/84 |
| Y | CN 1 103 817 C (UFZ LEIPZIGHALLE GMBH [DE]) 26 March 2003 (2003-03-26) * description; claim 1; figure 1 * | 7-9 | |
| A | CN 111 498 996 A (UNIV GUANGDONG PETROCHEM TECH) 7 August 2020 (2020-08-07) * claims 1, 5, 6, 7, 9 * | 1-9 | |
| A | EP 3 919 606 A1 (ALGAE & ALGAE TECH LTD [GB]) 8 December 2021 (2021-12-08) * paragraph [0001]; claim 4; figure 1 * | 1-9 | |
| | -/-- | | TECHNICAL FIELDS SEARCHED (IPC) C12M C12N |

The present search report has been drawn up for all claims

| Place of search | Date of completion of the search | Examiner |
|---|---|---|
| The Hague | 26 August 2025 | Guimarães, Bárbara |

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 17 7779

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BAHR MELANIE ET AL: "Microalgal-Biotechnology As a Platform for an Integral Biogas Upgrading and Nutrient Removal from Anaerobic Effluents", ENVIRONMENTAL SCIENCE & TECHNOLOGY, vol. 48, no. 1, 12 December 2013 (2013-12-12), pages 573-581, XP093301594, US ISSN: 0013-936X, DOI: 10.1021/es403596m Retrieved from the Internet: URL:https://pubs.acs.org/doi/pdf/10.1021/es403596m?ref=article_openPDF> * abstract * * figures 1, 7 * * page 103, column 2 * * page 104, column 1 * * page 104, column 2 * ----- | 1-6 | |
| A | MEIER L ET AL: "Photosynthetic $CO_2$ uptake by microalgae: An attractive tool for biogas upgrading", BIOMASS AND BIOENERGY, PERGAMON, AMSTERDAM, NL, vol. 73, 2 January 2015 (2015-01-02), pages 102-109, XP029198236, ISSN: 0961-9534, DOI: 10.1016/J.BIOMBIOE.2014.10.032 * abstract * * page 574, column 1 * * figure 1 * ----- | 1-6 | TECHNICAL FIELDS SEARCHED (IPC) |

The present search report has been drawn up for all claims

| Place of search | Date of completion of the search | Examiner |
|---|---|---|
| The Hague | 26 August 2025 | Guimarães, Bárbara |

EPO FORM 1503 03.82 (P04C01)

## ANNEX TO THE EUROPEAN SEARCH REPORT
## ON EUROPEAN PATENT APPLICATION NO.

EP 25 17 7779

This annex lists the patent family members relating to the patent documents cited in the above-mentioned European search report.
The members are as contained in the European Patent Office EDP file on
The European Patent Office is in no way liable for these particulars which are merely given for the purpose of information.

26-08-2025

| Patent document cited in search report | | | Publication date | Patent family member(s) | | | Publication date |
|---|---|---|---|---|---|---|---|
| EP 3441472 | A1 | | 13-02-2019 | NONE | | | |
| DE 3912418 | C1 | | 12-07-1990 | NONE | | | |
| CN 1103817 | C | | 26-03-2003 | AU | 8016198 | A | 08-12-1998 |
| | | | | BR | 9809114 | A | 01-08-2000 |
| | | | | CN | 1255946 | A | 07-06-2000 |
| | | | | WO | 9851814 | A1 | 19-11-1998 |
| CN 111498996 | A | | 07-08-2020 | NONE | | | |
| EP 3919606 | A1 | | 08-12-2021 | NONE | | | |

EPO FORM P0459

For more details about this annex : see Official Journal of the European Patent Office, No. 12/82